# DEMANDE DE BREVET EUROPEEN

(11) **EP 2 213 194 A2**
(43) Date de publication de la demande: **04.08.2010**
(21) Numéro de dépôt: 10151907.2
(22) Date de dépôt: 28.01.2010
(51) Int. Cl.: A45D 7/06, A61Q 5/00

(54) **Dispositif de chauffage de fibres kératiniques, procédé et kit correspondants**

(30) Priorité: 30.01.2009 FR 0950606
(71) Demandeur: L'Oréal, 75008 Paris (FR)
(72) Inventeur: Takahashi, Hiroshi, TOKYO 206-0802 (JP); De Boni, Maxime, TOKYO 162-0842 (JP)
(74) Mandataire: Casalonga, Axel

(57) **Abrégé**

La présente demande concerne un procédé de traitement de fibres kératiniques, dans lequel on expose les fibres kératiniques à un rayonnement infrarouge comprenant des longueurs d'onde se situant dans la gamme de longueurs d'onde allant de 700 à 1400 nanomètres, de préférence de 750 à 1400 nanomètres, et éventuellement des longueurs d'onde se situant dans la gamme de longueurs d'onde allant de 1400 nanomètres à 1 millimètre, de préférence de 1400 à 25000 nanomètres, et plus préférentiellement de 1400 à 15000 nanomètres.

La présente demande concerne aussi un dispositif de chauffage de fibres kératiniques, en particulier des cheveux, pour la mise en oeuvre du procédé selon l'invention, comprenant un ou plusieurs émetteurs d'un rayonnement infrarouge, dans lequel le rayonnement infrarouge comprend des longueurs d'onde comprises entre 700 et 1400 nanomètres, de préférence de 750 à 1400 nanomètres, et des longueurs d'onde comprises entre 15000 nanomètres et 1 millimètre, de préférence entre 15000 et 25000 nanomètres.

Elle concerne enfin un kit comprenant :
- un ou plusieurs dispositifs de chauffage de fibres kératiniques selon l'invention, et
- une ou plusieurs compositions de traitement des fibres kératiniques.

## Description

La présente invention a pour objet un dispositif de chauffage de fibres kératiniques, ainsi que le procédé de chauffage correspondant.

Il existe divers traitements appliqués aux fibres kératiniques humaines. Ces traitements peuvent avoir pour but d'en changer l'aspect. A titre d'exemples de procédé permettant de changer la couleur, on peut citer la coloration des fibres (coloration d'oxydation ou coloration directe), et la décoloration des fibres. A titre d'exemples de procédé permettant de modifier la forme des fibres, on peut citer la permanente et le défrisage ou décrêpage ou lissage, dans lesquels on peut, par exemple, mettre en contact les fibres avec une composition très fortement alcaline, comprenant des hydroxydes, avec pour résultat de transformer des ponts disulfures par lanthionisation. Ces traitements peuvent également avoir pour but de nettoyer les fibres kératiniques, de protéger, de préserver les fibres kératiniques, ou d'améliorer leurs propriétés cosmétiques, par exemple les traitements de conditionnement, ou les traitements de protection contre les ultraviolets.

Il est par ailleurs connu d'utiliser, lors de ces différents traitements cosmétiques, des dispositifs de chauffage des fibres kératiniques, par exemple un sèche-cheveux, un fer à cheveux ou des rouleaux chauffants.

Parmi les différents traitements cosmétiques, la modification durable de la forme des fibres est l'une des applications les plus développées. La tenue de la coiffure dans le temps, l'efficacité de la mise en forme en terme de bouclage et l'impact sur les fibres (notamment en terme de fragilisation ou de dégradation des fibres) sont des critères importants pour ce type de traitement, et en particulier pour les traitements utilisant des réducteurs (permanente et produits de lissage) ou des agents fortement alcalins (produits de défrisage). Des procédés associés à ces produits ont notamment été développés pour augmenter leur efficacité. Un exemple classique de procédé d'amélioration du traitement de fibres kératiniques est la permanente à chaud.

En comparaison avec un procédé classique de permanente à froid, la permanente à chaud présente l'avantage principal d'améliorer l'efficacité en terme de bouclage de la permanente et la tenue dans le temps de la coiffure. Cependant, comme les fibres kératiniques sont chauffées à une température relativement importante (jusqu'à 90-110°C) et comme la durée de chauffage est relativement longue (en moyenne entre 20 et 30 minutes pour l'étape de chauffage), les fibres kératiniques subissent une dégradation importante qui ne permet pas aux utilisateurs d'envisager une répétition d'un tel procédé ou bien encore une combinaison de ce procédé avec d'autres traitements comme la coloration ou la décoloration par exemple.

Il est connu des documents EP 1 661 481, JP 2006/087628, EP 1 579 782 ou JP 10108719 d'utiliser des infrarouges lointains pour chauffer des cheveux. Il est également connu du document US 2003/091602 d'utiliser des proches infrarouges et une composition de traitement capable d'être modifiée par les proches infrarouges.

Cependant, les produits et procédés décrits précédemment n'offrent des performances correctes que le jour du traitement. De plus, il y a de nombreux inconvénients qui ne sont pas compatibles avec les attentes des utilisateurs, par exemple :
- un niveau élevé de dégradation des fibres kératiniques, notamment en applications répétées ou en combinaison avec d'autres traitements tels que la coloration par oxydation,
- une tenue dans le temps insuffisante dans des conditions contraignantes (tension mécanique lors d'un brushing, shampoings répétés, exposition à la lumière),
- l'absence de rôle bénéfique sur l'état cosmétique, c'est-à-dire l'absence d'amélioration, lors du chauffage, de l'effet apporté par une composition de traitement précédemment appliquée sur les fibres kératiniques.

Le but de la présente invention est d'augmenter les performances des traitements des fibres kératiniques, notamment la tenue dans le temps et l'efficacité du traitement, tout en limitant la dégradation des fibres.

Ce but est atteint par la présente invention qui a pour objet un procédé de traitement de fibres kératiniques, en particulier des cheveux, dans lequel on expose les fibres kératiniques à un rayonnement dans le proche infrarouge, et éventuellement à un rayonnement dans l'infrarouge moyen et/ou lointain.

En particulier, le rayonnement proche infrarouge comprend des longueurs d'onde se situant dans la gamme de longueurs d'onde allant de 700 à 1400 nanomètres, et de préférence de 750 à 1400 nanomètres. Le rayonnement infrarouge moyen comprend des longueurs d'onde se situant dans la gamme de longueurs d'onde allant de 1400 nanomètres à 15000 nanomètres, et le rayonnement infrarouge lointain comprend des longueurs d'onde se situant dans la gamme de longueurs d'onde allant de 15000 nanomètre à 1 millimètre.

Ainsi, le procédé de traitement expose les fibres kératiniques à un rayonnement infrarouge comportant des longueurs d'onde se situant dans la gamme de longueurs d'onde allant de 700 à 1400 nanomètres, et de préférence de 750 à 1400 nanomètres. Le rayonnement infrarouge peut comprendre en outre des longueurs d'onde se situant dans la gamme de longueurs d'onde allant de 1400 nanomètres à 1 millimètre, de préférence de 1400 à 25000 nanomètres, et plus préférentiellement de 1400 à 15000 nanomètres.

L'invention a de même pour objet un dispositif de chauffage de fibres kératiniques, en particulier des cheveux, pour la mise en oeuvre du procédé selon l'invention, comportant un ou plusieurs émetteurs d'un rayonnement infrarouge comprenant des longueurs d'onde se situant dans la gamme de longueurs d'onde allant de 700 à 1400 nanomètres, de préférence de 750 à 1400 nanomètres, et des longueurs d'onde comprises entre 15000 nanomètres et 1 millimètre, de préférence de 15000 à 25000 nanomètres

L'invention concerne aussi un kit comprenant :
- un ou plusieurs dispositifs de chauffage de fibres kératiniques selon l'invention, et
- une ou plusieurs compositions de traitement des fibres kératiniques.

La composition de traitement des fibres kératiniques peut être choisie parmi une composition de coloration directe, une composition de coloration par oxydation, une composition réductrice de permanente, une composition oxydante de permanente, une composition de décoloration, une composition de conditionnement telle qu'un après-shampooing comme un soin rincé ou non-rincé, une composition de nettoyage telle qu'un shampooing, une composition de coiffage, une composition de protection contre les ultraviolets.

Il est à noter que dans ce qui va suivre, et à moins d'une autre indication, les bornes d'un domaine de valeurs sont comprises dans ce domaine.

Par ailleurs, on entend par « chauffage de fibres kératiniques » une élévation de la température des fibres traitées. Ce chauffage peut conduire éventuellement à une évaporation partielle ou complète d'une composition de traitement appliquée précédemment sur lesdites fibres, c'est-à-dire peut conduire à un séchage partiel ou complet des fibres kératiniques.

L'utilisation d'un rayonnement proche infrarouge permet de réduire de manière significative la durée de chauffage des fibres kératiniques, même avec une température faible, notamment grâce au chauffage efficace, par les proches infrarouges, à l'intérieur de la chevelure et des cheveux. En effet, par comparaison avec les infrarouges moyens ou lointains, les proches infrarouges présentent une profondeur de pénétration à l'intérieur de la chevelure et des cheveux qui est plus importante, et qui permet donc de ne pas agir uniquement en surface.

En particulier, l'utilisation d'un rayonnement proche infrarouge permet notamment d'avoir un chauffage rapide, même à faible température (c'est-à-dire à des températures comprises entre 25°C et 80°C, de préférence entre 25°C et 60°C), et d'obtenir une modification de la mise en forme des cheveux durable sans provoquer de dommages supplémentaires, notamment dus à une température trop élevée, c'est-à-dire supérieure à 80°C, ou même à 60°C.

Le rayonnement infrarouge utilisé lors du procédé de traitement selon l'invention présente de préférence un premier maximum d'intensité se situant à une longueur d'onde comprise dans la gamme de longueurs d'onde allant de 700 à 1400 nanomètres, de préférence de 750 à 1400 nanomètres. Ces gammes de longueurs d'onde appartiennent à la gamme des proches infrarouges. La présence de ce premier maximum d'intensité dans le rayonnement infrarouge indique que les proches infrarouges sont présents en quantité suffisante pour conférer au rayonnement infrarouge total les effets décrits précédemment. En particulier, on pourra choisir l'intensité de ce premier maximum de manière à ce que le chauffage effectif des fibres kératiniques par les proches infrarouges soit prépondérant dans le procédé de chauffage. Le rayonnement infrarouge pourra alors ne pas comprendre de longueur d'onde se situant dans la gamme de longueurs d'onde allant de 1400 nanomètres à 1 millimètre, de préférence de 1400 à 25000 nanomètres, et plus préférentiellement de 1400 à 15000 nanomètres. Alternativement, le rayonnement infrarouge pourra comprendre des infrarouges moyens et/ou lointains, c'est-à-dire à des longueurs d'onde allant de 1400 nanomètres à 1 millimètre, qui ne présentent pas de maximum d'intensité dans cette gamme de longueurs d'onde.

Le rayonnement infrarouge peut également présenter un ou plusieurs autres maxima d'intensité se situant à une longueur d'onde comprise dans la gamme de longueurs d'onde allant de 1400 nanomètres à 1 millimètre, de préférence de 1400 à 25000 nanomètres, et plus préférentiellement de 1400 à 15000 nanomètres. La gamme de longueurs d'onde allant de 1400 nanomètres à 1 millimètre correspond, comme indiqué ci-dessus, aux infrarouges moyens et lointains. La présence d'au moins un deuxième maximum dans cette gamme de longueurs d'onde indique que l'étape de chauffage n'est pas réalisée uniquement par les proches infrarouges. Les infrarouges moyens et/ou lointains participent également au procédé de chauffage, notamment par une action en surface. La présence de ce ou ces maxima d'intensité supplémentaires indique également que les infrarouges moyens et/ou lointains ne sont pas uniquement une conséquence de l'émission des proches infrarouges, mais sont volontairement présents dans le rayonnement infrarouge.

Dans ce cas, l'intensité du ou des maxima d'intensité supplémentaires peut être inférieure à celle du premier maximum.

Selon un mode de mise en oeuvre, le rayonnement infrarouge comprend des longueurs d'onde comprises entre 700 et 1400 nanomètres, de préférence entre 750 et 1400 nanomètres, et des longueurs d'onde comprises entre 1400 et 15000 nanomètres, et de préférence entre 1400 et 3000 nanomètres. Dans ce mode de mise en oeuvre, les proches infrarouges sont associés à des infrarouges moyens.

Selon un autre mode de mise en oeuvre, le rayonnement infrarouge comprend des longueurs d'onde comprises entre 700 et 1400 nanomètres, de préférence entre 750 et 1400 nanomètres, et des longueurs d'onde comprises entre 15000 nanomètres et 1 millimètre, de préférence de 15000 à 25000 nanomètres. Dans ce mode de mise en oeuvre, les proches infrarouges sont associés à des infrarouges lointains.

L'intensité du rayonnement compris dans la gamme de longueurs d'onde allant de 1400 nanomètres à 1 millimètre, de préférence de 1400 à 25000 nanomètres, peut représenter 0% à 60%, de préférence 10% à 40%, de l'intensité du rayonnement infrarouge. L'intensité du rayonnement proche infrarouge est choisie de préférence supérieure à celle des rayonnements infrarouges moyens et/ou lointains. Cependant, dans la mesure où la gamme de longueurs d'onde des proches infrarouges est très étroite par rapport à la gamme de longueurs d'onde de l'infrarouge moyen et lointain, la somme des intensités des rayonnements infrarouges moyens et lointains peut être éventuellement supérieure à l'intensité du rayonnement des proches infrarouges.

Dans un mode de réalisation, le dispositif de chauffage peut comprendre un premier moyen d'émission émettant des infrarouges se situant dans la gamme de longueurs d'onde allant de 700 à 1400 nanomètres, de préférence entre 750 et 1400 nanomètres, et un deuxième moyen d'émission émettant des infrarouges se situant dans la gamme de longueurs d'onde allant de 15000 nanomètres à 1 millimètre, de préférence de 15000 à 25000 nanomètres. En particulier, le dispositif de chauffage comprend deux moyens permettant d'émettre d'une part des proches infrarouges et d'autre part des infrarouges lointains. On comprend ainsi que le rayonnement infrarouge total puisse présenter deux maxima d'intensité situés chacun dans la gamme d'émission du moyen correspondant. Par ailleurs, l'utilisation de deux moyens distincts permet de modifier simplement la proportion relative de proches infrarouges et des infrarouges lointains dans le rayonnement total, notamment en modifiant l'alimentation électrique des deux moyens, et donc leur intensité de rayonnement. Il est ainsi possible d'ajuster la proportion des différents infrarouges pour obtenir l'effet voulu, tout en limitant les dommages sur les fibres.

Le dispositif de chauffage peut se présenter sous la forme d'une lampe infrarouge, d'un sèche-cheveux, d'un peigne chauffant, d'une brosse chauffante, d'un fer à cheveux, d'un casque de coiffure ou d'un rouleau chauffant.

Le dispositif selon l'invention peut également comprendre un minuteur programmable capable d'alimenter l'émetteur pendant une durée déterminée. En particulier, la durée déterminée est fonction de l'inertie thermique de l'émetteur. Le minuteur permet de déterminer la durée d'exposition aux infrarouges, et donc l'efficacité du procédé de chauffage. Cependant, le dispositif peut comprendre des moyens d'émission présentant une inertie thermique très différente, c'est-à-dire qu'un moyen peut nécessiter un certain temps pour commencer à émettre des infrarouges alors qu'un autre moyen émettra plus rapidement avec une alimentation électrique identique. De même, à la fin du procédé, un moyen pourra nécessiter un certain temps pour ne plus émettre d'infrarouges alors qu'un autre moyen cessera d'émettre rapidement. Lorsque le dispositif rayonne des proches infrarouges et des infrarouges lointains, le minuteur peut commander ces différents rayonnements éventuellement de manière indépendante de façon à obtenir une exposition pendant la durée souhaitée au rayonnement infrarouge voulu.

Selon un mode de réalisation, le minuteur peut également commander les moyens d'émission du dispositif afin d'exposer les fibres kératiniques d'abord aux proches infrarouges puis aux infrarouges lointains, ou inversement. On peut alors découpler l'action des deux types d'infrarouges, et exposer les fibres kératiniques à ces deux types d'infrarouges pendant des durées différentes.

L'intensité du rayonnement infrarouge peut être ajustée de manière à ce que la température des fibres kératiniques soit comprise entre 25°C et 80°C, de préférence entre 25°C et 60°C. L'invention permet alors d'obtenir un chauffage tout en limitant la température des cheveux, et donc tout en limitant la fragilisation ou la dégradation des cheveux.

Le procédé selon l'invention peut comprendre une étape dans laquelle on applique également une ou plusieurs compositions de traitement sur les fibres avant, pendant et/ou après le chauffage des fibres. En particulier, l'utilisation d'une étape de chauffage par le rayonnement infrarouge selon l'invention, peut également améliorer l'efficacité de la composition de traitement, notamment en permettant de renforcer sa pénétration dans les fibres ou d'activer ses propriétés.

La composition de traitement peut être choisie parmi une composition de coloration directe, une composition de coloration par oxydation, une composition réductrice de permanente, une composition oxydante de permanente, une composition de décoloration, une composition de conditionnement telle qu'un après-shampooing comme un soin rincé ou non-rincé, une composition de nettoyage telle qu'un shampooing, une composition de coiffage, une composition de protection contre les ultraviolets.

Le procédé selon l'invention peut également comprendre :
a) une étape de mise sous tension des fibres kératiniques, puis
b) une étape d'application sur les fibres kératiniques d'une composition réductrice, pour réduire les liaisons disulfures de la kératine, puis, après un éventuel rinçage,
c) une étape de chauffage des fibres kératiniques par exposition à un rayonnement infrarouge comprenant des longueurs d'onde se situant dans la gamme de longueurs d'onde allant de 700 à 1400 nanomètres, de préférence de 750 à 1400 nanomètres, et éventuellement des longueurs d'onde se situant dans la gamme de longueurs d'onde allant de 1400 nanomètres à 1 millimètre, de préférence de 1400 à 25000 nanomètres, et plus préférentiellement de 1400 à 15000 nanomètres, puis
d) une étape de fixation par oxydation, pour reformer lesdites liaisons disulfures, par application sur les fibres kératiniques d'une composition oxydante.

Cet exemple de mise en oeuvre de l'invention dans le cadre d'une permanente permet d'améliorer l'efficacité de bouclage de la mise en forme, tout en limitant la température d'exposition des fibres kératiniques.

Préférentiellement, on chauffe les fibres pendant 1 à 45 minutes, de préférence pendant 10 à 30 minutes.

L'invention concerne également un kit comprenant un ou plusieurs dispositifs de chauffage de fibres kératiniques selon l'invention, et une ou plusieurs compositions de traitement des fibres kératiniques. La ou les compositions sont choisies parmi une composition de coloration directe, une composition de coloration par oxydation, une composition réductrice de permanente, une composition oxydante de permanente, une composition de décoloration, une composition de conditionnement telle qu'un après-shampooing comme un soin rincé ou non-rincé, une composition de nettoyage telle qu'un shampooing, une composition de coiffage, une composition de protection contre les ultraviolets.

La composition peut se présenter sous différentes formes, telles qu'une lotion, une crème, un gel ou sous toute autre forme appropriée pour une application sur des fibres kératiniques.

La composition est conditionnée dans un compartiment ou récipient ou dispositif distinct, accompagné, éventuellement, de moyens d'application appropriés, tels que les pinceaux, les brosses, les éponges. Elle peut également être conditionnée sous pression en flacon aérosol en présence d'un agent propulseur et former une mousse.

Les exemples suivants servent à illustrer l'invention sans toutefois présenter un caractère limitatif.

### EXEMPLES DE MISE EN OEUVRE :

### EXEMPLES 1 à 4 :

On a préparé les compositions suivantes (teneurs exprimées en pourcentage de matière active) :
Composition (i) (composition réductrice) :

| | |
|---|---|
| Acide thioglycolique | 6,7 |
| Bicarbonate d'ammonium | 2,7 |
| Pentasodium pentetate (à 40% en solution aqueuse) | 0,4 |
| Ammoniaque (à 20% en solution aqueuse) | q.s.p. pH=8,7 |
| Eau déminéralisée | q.s.p. 100 |

Composition (ii) (composition oxydante) :

| | |
|---|---|
| Bromure de sodium | 8 |
| Phosphate de trisodium | 0,4 |
| Phosphate de sodium | 0,4 |
| Acide citrique | q.s.p. pH=7,5 |
| Eau déminéralisée | qsp 100 |

### Exemple 1 (invention) :

La composition (i) est appliquée pendant 15 minutes sur 2,5g de mèches de cheveux naturels de type japonais précédemment enroulés sur un rouleau de permanente de 1,7 cm de diamètre. Après un temps de pause, les cheveux sont rincés.

Les cheveux sont ensuite soumis à un rayonnement émis par une lampe infrarouge (QIR100V, 500W/D de USHIO) d'une puissance de 500W, avec une intensité maximale à une longueur d'onde de 1200nm et une intensité de 3,2 W/(CM².sr.µm), pendant 30 minutes. La lampe fournit un large spectre infrarouge allant du proche infrarouge à l'infrarouge lointain, avec un maximum d'intensité situé dans le proche infrarouge. L'intensité du rayonnement est ajustée de manière à ce que la température des cheveux ne dépasse pas 50°C.

La composition (ii) est ensuite appliquée pendant 10 minutes. Après un temps de pause, les cheveux sont retirés du rouleau à permanente, rincés et séchés.

### Exemple 2 (comparatif) :

La composition (i) est appliquée pendant 15 minutes sur 2,5g de mèches de cheveux naturels de type japonais précédemment enroulés sur un rouleau de permanente de 1,7 cm de diamètre. Après un temps de pause, les cheveux sont rincés et la composition (ii) est appliquée pendant 10 minutes. Les cheveux sont alors retirés du rouleau à permanente, rincés et séchés.

### Exemple 3 (comparatif) :

La composition (i) est appliquée pendant 15 minutes sur 2,5g de mèches de cheveux naturels de type japonais. Après un temps de pause, les cheveux sont rincés puis enroulés sur un rouleau de permanente de 1,7 cm de diamètre.

Le rouleau à cheveux est alors chauffé à 90°C pendant 30 minutes (machine Digital Perm, modèle ODIS-2 de OOHIRO).

Après l'étape de chauffage, la composition (ii) est appliquée pendant 10 minutes. Les cheveux sont alors retirés du rouleau à permanente, rincés et séchés.

### Exemple 4 (comparatif) :

La composition (i) est appliquée pendant 15 minutes sur 2,5g de mèches de cheveux naturels de type japonais. Après un temps de pause, les cheveux sont rincés puis enroulés sur un rouleau de permanente de 1,7 cm de diamètre.

Le rouleau à cheveux est alors chauffé à 50°C pendant 30 minutes (machine Digital Perm, modèle ODIS-2 de OOHIRO). Il convient de noter qu'il faut plus de 60 minutes pour sécher les cheveux dans le cas d'un rouleau chauffant à 50°C.

Après l'étape de chauffage, la composition (ii) est appliquée pendant 10 minutes. Les cheveux sont alors retirés du rouleau à permanente, rincés et séchés.

### Evaluation de la permanente :

Un test de tenue des boucles est réalisé sur les cheveux permanentés selon les exemples 1 à 4 décrits précédemment. En particulier, les mèches de cheveux sont maintenues droites sous tension, pendant 5 heures, avec une humidité relative d'environ 100%. La tenue des boucles est évaluée par comparaison de la forme des cheveux avant et après le test.

Pour évaluer l'impact du procédé de permanente sur les cheveux, l'efficacité de bouclage après des applications répétées (3 permanentes) a été aussi mesurée.

Les résultats sont donnés dans le tableau ci-dessous :

| | Efficacité de bouclage après une permanente | Tenue des boucles | Efficacité de bouclage après trois permanentes |
|---|---|---|---|
| Exemple 1 | Très bonne | Très bonne | Bonne |
| (invention) | | | |
| Exemple 2 | Bonne | Faible | Faible |
| (comparatif) | | | |
| Exemple 3 | Très bonne | Très bonne | Très faible |
| (comparatif) | | | |
| Exemple 4 | Bonne | Faible | Très faible |
| (comparatif) | | | |

### EXEMPLES 5 à 8 :

On a préparé les compositions suivantes (teneurs exprimées en pourcentage de matière active) :
Composition (iii) (composition réductrice) :

| | |
|---|---|
| Cystéine | 6,06 |
| Bicarbonate d'ammonium | 2,8 |
| Pentasodium pentetate (à 40% en solution aqueuse) | 0,4 |
| Monoéthanolamine | 2,8 |
| Eau déminéralisée | q.s.p. 100 |

Composition (iv) (composition réductrice) :

| | |
|---|---|
| N-acetyl-Cystéine | 8,15 |
| Bicarbonate d'ammonium | 2,8 |
| Pentasodium pentetate (à 40% en solution aqueuse) | 0,4 |
| Monoéthanolamine | 2,8 |
| Eau déminéralisée | q.s.p. 100 |

Pour ces exemples, des cheveux naturels de type japonais ont été décolorés en utilisant un produit commercial de décoloration de cheveux (PLATIFIZTM COMPACT de L'OREAL). La décoloration a eu lieu à 40°C pendant 30 minutes.

### Exemple 5 (invention) :

La composition (iii) est appliquée pendant 15 minutes sur 1g de mèches de cheveux décolorés de type japonais, précédemment enroulés sur un rouleau de permanente de 1,7 cm de diamètre. Après un temps de pause, les cheveux sont rincés.

Puis, les cheveux sont soumis à un rayonnement émis par une lampe infrarouge (QIR100V, 500W/D de USHIO, d'une puissance de 500W, avec une intensité maximale à une longueur d'onde de 1200nm et une intensité de 3,2 W/(CM².sr.µm)) pendant 30 minutes. L'intensité du rayonnement est ajustée de manière à ce que la température des cheveux ne dépasse pas 50°C.

La composition (ii) est ensuite appliquée pendant 10 minutes. Après un temps de pause, les cheveux sont retirés du rouleau à permanente, rincés et séchés.

### Exemple 6 (comparatif) :

La composition (iii) est appliquée pendant 15 minutes sur 1g de mèches de cheveux décolorés de type japonais, précédemment enroulés sur un rouleau de permanente de 1,7 cm de diamètre. Après un temps de pause, les cheveux sont rincés puis la composition (ii) est appliquée pendant 10 minutes. Après un temps de pause, les cheveux sont retirés du rouleau à permanente, rincés à nouveau et séchés.

### Exemple 7 (invention) :

La composition (iv) est appliquée pendant 15 minutes sur 1g de mèches de cheveux décolorés de type japonais, précédemment enroulés sur un rouleau de permanente de 1,7 cm de diamètre. Après un temps de pause, les cheveux sont rincés.

Puis, les cheveux sont soumis à un rayonnement émis par une lampe infrarouge (QIR100V, 500W/D de USHIO, d'une puissance de 500W, avec une intensité maximale à une longueur d'onde de 1200nm et une intensité de 3,2 W/(cm².sr.µm)) pendant 30 minutes. L'intensité du rayonnement est ajustée de manière à ce que la température des cheveux ne dépasse pas 50°C.

La composition (ii) est ensuite appliquée pendant 10 minutes. Après un temps de pause, les cheveux sont retirés du rouleau à permanente, rincés et séchés.

### Exemple 8 (comparatif) :

La composition (iv) est appliquée pendant 15 minutes sur 1g de mèches de cheveux décolorés de type japonais, précédemment enroulés sur un rouleau de permanente de 1,7 cm de diamètre. Après un temps de pause, les cheveux sont rincés puis la composition (ii) est appliquée pendant 10 minutes. Après un temps de pause, les cheveux sont retirés du rouleau à permanente, rincés à nouveau et séchés. Le degré de bouclage est faible.

### Evaluation de la permanente :

Le test de tenue des boucles est réalisé sur les cheveux permanentés selon les exemples 5 à 8 décrits précédemment.

Les résultats sont donnés dans le tableau ci-dessous :

| | Efficacité de bouclage après une permanente | Tenue des boucles |
|---|---|---|
| Exemple 5 | Très bonne | Très bonne |
| (invention) | | |
| Exemple 6 | Très bonne | Faible |
| (comparatif) | | |
| Exemple 7 | Bonne | Bonne |
| (invention) | | |
| Exemple 8 | Bonne | Très faible |
| (comparatif) | | |

### EXEMPLE 9

On a préparé la composition suivante (teneurs exprimées en pourcentage de matière active) :

| | |
|---|---|
| Acide thioglycolique | 1,5 |
| Cystéine | 0,15 |
| Bicarbonate d'ammonium | 2,7 |
| Pentasodium pentetate (à 40% en solution aqueuse) | 0,4 |
| Ammoniaque (à 20% en solution aqueuse) | q.s.p. pH=8 |
| Eau déminéralisée | q.s.p. 100 |

La composition est appliquée pendant 15 minutes sur 1g de mèches de cheveux décolorés de type japonais, précédemment enroulés sur un rouleau de permanente de 1,7 cm de diamètre. Durant le temps de pause, les cheveux sont soumis à un rayonnement émis par une lampe infrarouge (QIR100V 500W/D de USHIO, d'une puissance de 500W, avec une intensité maximale à une longueur d'onde de 1200nm et une intensité de 3,2 W/(cm².sr.µm)).

La même composition est également appliquée sur 1g de mèches de cheveux décolorés de type japonais, pendant 15 minutes, mais sans rayonnement infrarouge.

Après un temps de pause, les cheveux sont rincés puis la composition (ii) est appliquée pendant 10 minutes. Les cheveux sont alors retirés du rouleau à permanente, rincés et séchés.

On constate, dans le cas des cheveux soumis à un rayonnement avec un maximum d'intensité dans le proche infrarouge, une efficacité de bouclage meilleure que dans le cas où les cheveux ne sont pas soumis à un rayonnement infrarouge.

### EXEMPLE 10

On a préparé la composition suivante (teneurs exprimées en pourcentage de matière active) :

| | |
|---|---|
| p-phenylène diamine | 0,65 |
| Resorcinol | 0,66 |
| Hydroxyéthylcellulose (720.000) | 0,72 |
| Décylpolyglucoside | 9,0 |
| Alcool benzylique | 4,0 |
| Conservateurs | 0,06 |
| Ammoniaque | 2 |
| Eau déminéralisée | q.s.p.100 |

La composition est mélangée, en masse sur masse, avec 20 volumes de solution oxydante, puis appliquée pendant 30 minutes sur 1g de mèches de cheveux (90% blancs) de type caucasien. Durant le temps de pause, les cheveux sont soumis à un rayonnement émis par une lampe infrarouge (QIR100V 500W/D de USHIO, d'une puissance de 500W, avec une intensité maximale à une longueur d'onde de 1200nm et une intensité de 3,2 W/(cm².sr.µm)).

La même composition est également appliquée sur 1g de mèches de cheveux (90% blancs) de type caucasien, pendant 30 minutes, mais sans rayonnement infrarouge.

Après un temps de pause, les cheveux sont rincés et séchés.

On constate, dans le cas des cheveux soumis à un rayonnement avec un maximum d'intensité dans le proche infrarouge, une nuance verte beaucoup plus profonde que dans le cas où les cheveux ne sont pas soumis à un rayonnement infrarouge.

### EXEMPLE 11

On a préparé la composition suivante (teneurs exprimées en pourcentage de matière active) :

| | |
|---|---|
| p-aminophenol | 0,65 |
| 1-β-hydroxyéthyloxy-2,4-diaminobenzène | 1,45 |
| Hydroxyéthylcellulose (720.000) | 0,72 |
| Décylpolyglucoside | 9,0 |
| Alcool benzylique | 4,0 |
| Conservateurs | 0,06 |
| Ammoniaque | 2 |
| Eau déminéralisée | q.s.p.100 |

La composition est mélangée, en masse sur masse, avec 20 volumes de solution oxydante, puis appliquée pendant 30 minutes sur 1g de mèches de cheveux (90% blancs) de type caucasien. Durant le temps de pause, les cheveux sont soumis à un rayonnement émis par une lampe infrarouge (QIR100V 500W/D de USHIO, d'une puissance de 500W, avec une intensité maximale à une longueur d'onde de 1200nm et une intensité de 3,2 W/(cm².sr.µm)).

La même composition est également appliquée sur 1g de mèches de cheveux (90% blancs) de type caucasien, pendant 30 minutes, mais sans rayonnement infrarouge.

Après un temps de pause, les cheveux sont rincés et séchés.

On constate, dans le cas des cheveux soumis à un rayonnement avec un maximum d'intensité dans le proche infrarouge, une nuance roux cuivré plus intense que dans le cas où les cheveux ne sont pas soumis à un rayonnement infrarouge.

## Revendications

1. Procédé de traitement de fibres kératiniques, en particulier des cheveux, comprenant :
a) une étape de mise sous tension des fibres kératiniques, puis
b) une étape d'application sur les fibres kératiniques d'une composition réductrice, pour réduire les liaisons disulfures de la kératine, puis, après un éventuel rinçage,
c) une étape de chauffage des fibres kératiniques par exposition à un rayonnement infrarouge comprenant des longueurs d'onde se situant dans la gamme de longueurs d'onde allant de 700 à 1400 nanomètres, de préférence de 750 à 1400 nanomètres, puis
d) une étape de fixation par oxydation, pour reformer lesdites liaisons disulfures, par application sur les fibres kératiniques d'une composition oxydante.

2. Procédé de traitement selon la revendication 1 dans lequel le rayonnement infrarouge présente un maximum d'intensité se situant à une longueur d'onde comprise dans la gamme de longueurs d'onde allant de 700 à 1400 nanomètres, de préférence de 750 à 1400 nanomètres.

3. Procédé de traitement selon la revendication 1 ou 2 dans lequel le rayonnement infrarouge ne comprend pas de longueur d'onde se situant dans la gamme de longueurs d'onde allant de 1400 nanomètres à 1 millimètre, de préférence de 1400 à 25000 nanomètres, et plus préférentiellement de 1400 à 15000 nanomètres.

4. Procédé de traitement selon la revendication 1 ou 2 dans lequel le rayonnement infrarouge comprend également des longueurs d'onde se situant dans la gamme de longueurs d'onde allant de 1400 nanomètres à 1 millimètre, de préférence de 1400 à 25000 nanomètres, et plus préférentiellement de 1400 à 15000 nanomètres.

5. Procédé de traitement selon la revendication 4 dans lequel le rayonnement infrarouge ne présente pas de maximum d'intensité dans la gamme de longueurs d'onde allant de 1400 nanomètres à 1 millimètre, de préférence de 1400 à 25000 nanomètres, et plus préférentiellement de 1400 à 15000 nanomètres.

6. Procédé de traitement selon la revendication 4 dans lequel le rayonnement infrarouge présente un ou plusieurs maxima d'intensité se situant à une longueur d'onde comprise dans la gamme de longueurs d'onde allant de 1400 nanomètres à 1 millimètre, de préférence de 1400 à 25000 nanomètres, et plus préférentiellement de 1400 à 15000 nanomètres.

7. Procédé de traitement selon l'une des revendications 4 à 6 dans lequel le rayonnement infrarouge comprend des longueurs d'onde comprises entre 700 et 1400 nanomètres, de préférence de 750 à 1400 nanomètres, et des longueurs d'onde comprises entre 15000 nanomètres et 1 millimètre, de préférence de 15000 à 25000 nanomètres.

8. Procédé de traitement selon l'une des revendications précédentes dans lequel l'intensité du rayonnement compris dans la gamme de longueurs d'onde allant de 1400 nanomètres à 1 millimètre, de préférence de 1400 à 25000 nanomètres, représente de 0% à 60%, de préférence de 10% à 40%, de l'intensité du rayonnement infrarouge.

9. Procédé selon l'une des revendications précédentes dans lequel l'intensité du rayonnement infrarouge du dispositif est ajustée de manière à ce que la température des fibres kératiniques soit comprise entre 25°C et 80°C, de préférence entre 25°C et 60°C.

10. Procédé selon l'une des revendications précédentes dans lequel on chauffe les fibres pendant 1 à 45 minutes, de préférence pendant 10 à 30 minutes.

11. Utilisation d'un dispositif de chauffage de fibres kératiniques, en particulier des cheveux, pour la mise en oeuvre du procédé selon l'une quelconque des revendications précédentes, comprenant un ou plusieurs émetteurs d'un rayonnement infrarouge, **caractérisé en ce que** le rayonnement infrarouge comprend des longueurs d'onde comprises entre 700 et 1400 nanomètres, de préférence entre 750 et 1400 nanomètres, et des longueurs d'onde comprises entre 15000 nanomètres et 1 millimètre, de préférence entre 15000 et 25000 nanomètres.

12. Utilisation selon la revendication précédente dans lequel le dispositif comprend un premier moyen d'émission émettant des infrarouges se situant dans la gamme de longueurs d'onde allant de 700 à 1400 nanomètres, de préférence de 750 à 1400 nanomètres, et un deuxième moyen d'émission émettant des infrarouges se situant dans la gamme de longueurs d'onde allant de 15000 nanomètres et 1 millimètre, de préférence de 15000 à 25000 nanomètres.

13. Utilisation selon l'une des revendications 11 à 12 dans lequel le dispositif se présente sous la forme d'une lampe infrarouge, d'un sèche-cheveux, d'un peigne chauffant, d'une brosse chauffante, d'un fer à cheveux, d'un casque de coiffure ou d'un rouleau chauffant.

14. Kit comprenant :
- un ou plusieurs dispositifs de chauffage de fibres kératiniques comportant un ou plusieurs émetteurs d'un rayonnement infrarouge, le rayonnement infrarouge comprenant des longueurs d'onde comprises entre 700 et 1400 nanomètres, de préférence entre 750 et 1400 nanomètres, et des longueurs d'onde comprises entre 15000 nanomètres et 1 millimètre, de préférence entre 15000 et 25000 nanomètres, et
- une ou plusieurs compositions réductrices de permanente et une ou plusieurs compositions oxydantes de permanente.
